(19) Europäisches Patentamt
European Patent Office
Office européen des brevets

(11) EP 0 632 718 B1

(12) **EUROPÄISCHE PATENTSCHRIFT**

(45) Veröffentlichungstag und Bekanntmachung des Hinweises auf die Patenterteilung:
**06.05.1998 Patentblatt 1998/19**

(21) Anmeldenummer: **94901872.5**

(22) Anmeldetag: **25.11.1993**

(51) Int. Cl.$^6$: **A61K 7/50**

(86) Internationale Anmeldenummer:
**PCT/EP93/03304**

(87) Internationale Veröffentlichungsnummer:
**WO 94/16676 (04.08.1994 Gazette 1994/18)**

(54) **HAAR- UND KÖRPERREINIGUNGSMITTEL**
HAIR AND BODY WASHING PRODUCT
PRODUIT D'HYGIENE CAPILLAIRE ET DE SOINS CORPORELS

(84) Benannte Vertragsstaaten:
**DE ES FR GB IT**

(30) Priorität: **26.01.1993 DE 4301994**

(43) Veröffentlichungstag der Anmeldung:
**11.01.1995 Patentblatt 1995/02**

(73) Patentinhaber:
**Wella Aktiengesellschaft**
**64274 Darmstadt (DE)**

(72) Erfinder:
- **BIMCZOK, Rudolf, Dr.**
  **D-64342 Seeheim (DE)**
- **STIEHM, Thomas, Dr.**
  **D-64846 Gross-Zimmern (DE)**
- **GROHE, Viola**
  **D-64367 Mühltal (DE)**

(56) Entgegenhaltungen:
**EP-A- 0 236 677** **EP-A- 0 358 216**
**EP-A- 0 371 339** **EP-A- 0 490 041**
**EP-A- 0 531 650**

- **SEIFEN, öLE, FETTE, WACHSE Bd. 116, Nr. 2 , 1. Februar 1990 , AUGSBURG, DE Seiten 60 - 68 BEHLER AT AL. 'Neue Verdickungsmittel für Tensidformulierungen'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 632 718 B1

Printed by Xerox (UK) Business Services
2.16.3/3.4

## Beschreibung

Gegenstand der vorliegenden Erfindung ist ein Haar- und Körperreinigungsmittel auf der Basis einer Kombination eines bestimmten Tensidgemisches mit bestimmten stickstoffreien Verdickern, das sich durch eine besonders gute Hautvertraglichkeit auszeichnet.

Haar- und Körperreinigungsmittel enthalten als Hauptbestandteil Tenside. An diese Tenside werden eine Vielzahl von Anforderungen gestellt. So sollen diese Tenside einerseits ein hohes Schaumbildungsvermögen und eine gute Reinigungswirkung besitzen und andererseits eine gute Augen-, Haut- und Schleimhautverträglichkeit aufweisen sowie biologisch abbaubar sein.

Es wurden bereits eine Vielzahl von Versuchen unternommen, Haar- und Körperreinigungsmittel zur Verfügung zu stellen, die sowohl eine gute Reinigungswirkung besitzen als auch hervorragend hautverträglich sind.

Kein bisher zur Verfügung gestelltes Haar- und Körperreinigungsmittel konnte die vorstehend genannten Anforderungen voll befriedigen.

Es bestand daher die Aufgabe, ein Haar- und Körperreinigungsmittel zur Verfügung zustellen, das eine gute Reinigungswirkung mit einer ausgezeichneten Hautverträglichkeit verbindet.

Hierzu wurde nunmehr gefunden, das die vorstehende Aufgabe in hervorragender Weise mit einem Mittel gelöst wird, welches eine Kombination eines Alkylethersulfats mit einem ethoxylierten Sulfobernsteinsäurehalbesters und einem dritten Tensid, das ausgewählt ist aus Alkylethercarboxylaten und Alkylpolyglycosiden,und einem stickstoffreien Verdicker enthält.

Gegenstand der vorliegenden Erfindung ist daher ein Haar- und Körperreinigungsmittel, welches dadurch gekennzeichnet ist, daß es

(A) 1 bis 28 Gewichtsprozent mindestens eines Alkali-oder Erdalkalisalzes eines mit 1 bis 10 Ethylenoxideinheiten ethoxylierten $C_{10}$- bis $C_{18}$-Alkylethersulfats,

(B) 1 bis 28 Gewichtsprozent mindestens eines ethoxylierten Sulfobernsteinsäurehalbesters der allgemeinen Formel

$$R^1(OCH_2CH_2)_mO-\underset{\underset{O}{\|}}{C}-CH_2-\underset{\underset{SO_3M}{|}}{CH}-COOM \qquad (I),$$

wobei $R^1$ einen $C_{10}$- bis $C_{18}$-Alkylrest bedeutet, M ein Alkali- oder Erdalkalikation darstellt und m eine ganze Zahl von 1 bis 10 bedeutet,

(C) 1 bis 28 Gewichtsprozent mindestens eines Tensids, welches ausgewählt ist aus

- einem Alkylethercarboxylat der allgemeinen Formel

$$R^2(OCH_2CH_2)_nOCH_2COOM \qquad (II),$$

wobei $R^2$ einen $C_{10}$- bis $C_{18}$-Alkylrest bedeutet, M ein Alkali- oder Erdalkalikation darstellt und n eine ganze Zahl von 1 bis 20 bedeutet, und

- einem Alkylpolyglucosid der allgemeinen Formel

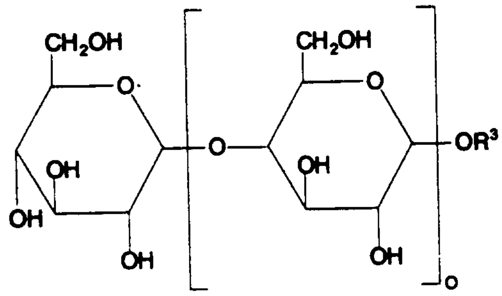

(III),

wobei $R^3$ einen linearen oder verzweigten, gesättigten oder ungesättigten $C_8$- bis $C_{18}$-Alkylrest, vorzugsweise einen $C_{10}$-Alkylrest, darstellt und o eine ganze Zahl von 1 bis 5, vorzugsweise von 1 bis 3, bedeutet sowie

(D) 0,1 bis 5 Gewichtsprozent mindestens eines stickstoffreien Verdickers, der ausgewählt ist aus

- ethoxylierten Methylglucosediestern von $C_{11}$- bis $C_{20}$-Fettsäuren der allgemeinen Formel

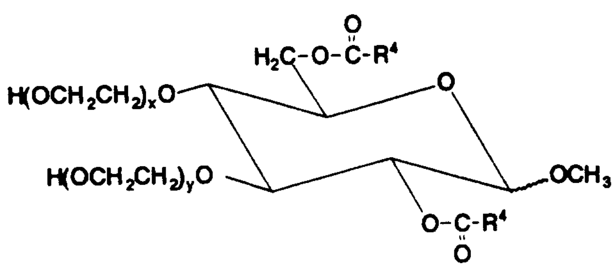

(IV),

wobei $R^4$ einen $C_{11}$- bis $C_{20}$-Alkylrest, bevorzugt einen $C_{17}$-Alkylrest, darstellt und die Summe von x und y 50 bis 200, vorzugsweise 120, ergibt,

- Polyethylenglykoldiestern der allgemeinen Formel

$$R^5-\overset{O}{\overset{\|}{C}}O(CH_2CH_2O)_z-\overset{O}{\overset{\|}{C}}-R^5 \qquad (V),$$

wobei $R^5$ einen $C_{11}$- bis $C_{20}$-Alkylrest, vorzugsweise einen $C_{17}$-Alkylrest, bedeutet und z eine ganze Zahl zwischen 70 und 200 darstellt und

- Gemischen aus a) mindestens einem Polyethylenglykolether von Fettsäurepartialglyceriden der allgemeinen Formel

$$R^6-\overset{O}{\overset{\|}{C}}-O-CH_2-\underset{OH}{\underset{|}{C}}H-CH_2(OCH_2CH_2)_pOH \qquad (VI),$$

wobei $R^6$ einen $C_{11}$- bis $C_{20}$-Alkylrest bedeutet und p eine ganze Zahl von 100 bis 300 darstellt und b) mindestens einem Polyethylenglykolether eines Fettsäurepartialglycerids der allgemeinen Formel (VI), wobei $R^6$ einen $C_{11}$- bis $C_{20}$-Alkylrest bedeutet und p eine ganze Zahl von 5 bis 10 darstellt,

enthält, wobei das Mittel außer den Komponenten (A), (B) und (C) keine weiteren Tenside enthält.

Unter einem Alkylrest werden in der vorliegenden Anmeldung lineare oder verzweigte, gesättigte oder ungesättigte Alkylreste verstanden.

Das erfindungsgemäße Haar- und Körperreinigungsmittel weist, bei vergleichbaren Wasch- und Reinigungseigenschaften, eine deutlich bessere Hautverträglichkeit auf als bekannte Haar- und Körperwaschmittel.

Das erfindungsgemäße Mittel enthält vorzugsweise 4 bis 10 Gewichtsprozent der Komponente (A), 2 bis 8 Gewichtsprozent der Komponente (B) und 1 bis 6 Gewichtsprozent der Komponente (C). Das Gewichtsverhältnis der Komponente (A) zur Komponente (B) sowie der Komponente (A) zur Komponente (C) beträgt in dem erfindungsgemäßen Mittel vorzugsweise jeweils 1:0,1 bis 1:1. Das Gewichtsverhältnis der Komponente (A) zur Komponente (D) beträgt in dem erfindungsgemäßen Mittel vorzugsweise 1:0,1 bis 1:0,8.

Das Tensidgemisch aus den Komponenten (A), (B) und (C) ist in dem erfindungsgemäßen Mittel vorzugsweise in einer Menge von 12 bis 30 Gewichtsprozent enthalten.

Das Alkylethersulfat der Komponente (A) ist bevorzugt mit 5 Ethylenoxideinheiten ethoxyliert. Der Alkylrest des Alkylethersulfats weist vorzugsweise 12 Kohlenstoffatome auf. Der ethoxylierte Sulfobernsteinsäurehalbester der Komponente (B) ist bevorzugt mit 3 Ethylenoxideinheiten ethoxyliert. Der Alkylrest des Sulfobernsteinsäurehalbesters weist bevorzugt 12 Kohlenstoffatome auf.

Als Komponente (C) enthält das erfindungsgemäße Haar- und Körperreinigungsmittel vorzugsweise ein Alkylethercarboxylat der allgemeinen Formel (II). Das Alkylethercarboxylat der Komponente (C) ist bevorzugt mit 10 Ethylenoxideinheiten ethoxyliert. Der Alkylrest des Alkylethercarboxylats weist bevorzugt 12 Kohlenstoffatome auf. Von den Alkylethercarboxylaten, die als Komponente (C) in dem erfindungsgemäßen Mittel enthalten sein können, ist ein mit 10 Ethylenoxideinheiten ethoxyliertes Laurylalkoholpolyethylenglykolethercarboxylat, das beispielsweise von der Firma Hüls AG, Marl/BRD unter der Handelsbezeichnung Marlinat® CM 105/80 vertrieben wird, besonders bevorzugt.

Von den Alkylpolyglucosiden, die als Komponente (C) in dem erfindungsgemäßen Mittel enthalten sein können, ist Decylglucosid bevorzugt, das beispielsweise in Form einer 55 prozentigen wäßrigen Lösung von der Firma Seppic, Paris/FR unter der Handelsbezeichnung Oramix® NS 10 vertrieben wird.

Von den stickstoffreien Verdickern, die als Komponente (D) in dem erfindungsgemäßen Mittel enthalten sein können, sind Gemische bestehend aus mindestens zwei Polyethylenglykolethern von Fettsäurepartialglyceriden a) und b) der allgemeinen Formel (VI) bevorzugt.

Als Gemisch von mindestens zwei Polyethylenglykolethern von Fettsäurepartialglyceriden a) und b) enthält das erfindungsgemäße Mittel besonders bevorzugt ein Gemisch aus den a) mit 200 Ethylenoxideinheiten ethoxylierten Polyethylenglykolethern der Mono- und Diglyceride des hydrierten Palmkernöls und b) den mit 7 Ethylenoxideinheiten ethoxylierten Polyethylenglykolethern der Kokosfettsäuremono- und -diglyceride (CTFA-Bezeichnung: PEG-200 Glyceryl Hydrogenated Palmitate and PEG-7 Glyceryl Cocoate), das beispielsweise von der Firma Rewo Chemische Werke GmbH, Steinau/BRD unter der Handelsbezeichnung Rewoderm® LI S 80 vertrieben wird.

Von den Methylglucosediestern, die als Komponente (D) in dem erfindungsgemäßen Mittel enthalten sein können, ist das mit 120 Einheiten Ethylenoxid ethoxylierte Methylglucosiddioleat, das beispielsweise von der Firma Amerchol, Edison/USA unter der Handelsbezeichnung Glucamate® DOE-120 vertrieben wird, bevorzugt.

Der Polyethylenglykoldiester der Komponente (D) ist bevorzugt mit 150 Ethylenoxideinheiten ethoxyliert. Von den Polyethylenglykoldiestern, die als Komponente (D) in dem erfindungsgemäßen Mittel enthalten sein können, ist das mit 150 Ethylenoxideinheiten ethoxylierte Polyethylenglykoldistearat besonders bevorzugt, das beispielsweise von der Firma Rewo Chemische GmbH, Steinau/BRD unter der Handelsbezeichnung Rewopal PEG 6000 DS vertrieben wird.

Der Wassergehalt des erfindungsgemäßen Mittels liegt vorzugsweise zwischen 65 und 85 Gewichtsprozent. Der pH-Wert des erfindungsgemäßen Mittels beträgt vorzugsweise 4 bis 7, besonders bevorzugt 5 bis 6, wobei die Einstellung des Mittels auf den gewünschten pH-Wert beispielsweise mit Zitronensäure, Phosphorsäure oder Natriumhydroxid erfolgen kann.

Im übrigen bestehen die hier beschriebenen Haar- und Körperreinigungsmittel aus einer Mischung der vorstehend genannten Komponenten (A), (B), (C) und (D) mit Wasser und den für kosmetische Reinigungsmittel üblichen Zusatzstoffen. Sie können dabei als klare oder getrübte Lösungen, als Emulsionen oder als Gele vorliegen. Bevorzugte Zubereitungsform ist die einer Lösung.

Als für kosmetische Reinigungsmittel übliche Zusatzstoffe kann das erfindungsgemäße Mittel beispielsweise Parfümöle in einer Menge von etwa 0,5 bis 5,0 Gewichtsprozent, Trübungsmittel, wie zum Beispiel Ethylenglykoldistearat, in einer Menge von etwa 0,5 bis 5,0 Gewichtsprozent, Perlglanzmittel, wie zum Beispiel ein Gemisch aus Fettsäuremonoalkyloamid/Ethylenglykoldistearat, in einer Menge von etwa 1,0 bis 10,0 Gewichtsprozent, bakterizide oder fungizide Wirkstoffe, wie zum Beispiel 2,4,4-Trichlor-2-hydroxy-diphenylether oder Methylchlorisothiazolinon, in einer Menge von etwa 0,01 bis 1,0 Gewichtsprozent, haar- und hautpflegende Wirkstoffe, wie zum Beispiel Fettsäureester, Fettalkohole, Chitosanderivate, Lanolinderivate und Proteinderivate, in einer Menge von 0,01 bis 3 Gewichtsprozent, Verdünnungsmittel, wie zum Beispiel 1,2-Propylenglykol oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa

0,1 bis 1,0 Gewichtsprozent, Lösungsvermittler, wie zum Beispiel ethoxyliertes, gegebenenfalls hydriertes Rizinusöl, in einer Menge von etwa 0,1 bis 1,0 Gewichtsprozent sowie Anfärbestoffe, wie zum Beispiel Fluorescein-Natriumsalz, in einer Menge von etwa 0,1 bis 1,0 Gewichtsprozent, enthalten.

Das erfindungsgemäße Haar- und Körperreinigungsmittel enthält bevorzugt 2 bis 20 Gewichtsprozent für kosmetische Reinigungsmittel übliche kosmetische Zusatzstoffe.

Die nachfolgenden Beispiele sollen den Gegenstand der vorliegenden Erfindung näher erläutern.

**Beispiele**

**Beispiel 1: Mildes Haar- und Körperreinigungsmittel**

| | |
|---|---|
| 7,00 g | mit 1 bis 4 Ethylenoxideinheiten ethoxyliertes Natriumlaurylethersulfat |
| 4,20 g | mit 1 bis 4 Ethylenoxideinheiten ethoxyliertes Dinatriumsalz des Laurylalkoholhalbesters der Sulfobernsteinsäure |
| 1,00 g | mit 10 Ethylenoxideinheiten ethoxyliertes Laurylalkoholpolyethylenglykolethercarboxylats |
| 1,00 g | mit 120 Einheiten Ethylenoxid ethoxyliertes Methylglucosiddioleat (Glucamate® DOE-120 der Firma Amercol, Edison/USA) |
| 2,00 g | Lauryldimethylammoniumhydroxypropylderivat des hydrolysierten Kollagens (Lamequat® L der Firma Henkel KGaA, Düsseldorf/BRD) |
| 0,60 g | Triethylenglykoldistearat |
| 0,20 g | Milchsäure |
| 0,40 g | Parfümöl |
| 83,60 g | Wasser |
| 100,00 g | |

**Beispiel 2: Mildes Haar- und Körperreinigungsmittel**

| | |
|---|---|
| 7,00 g | mit 1 bis 4 Ethylenoxideinheiten ethoxyliertes Natriumlaurylethersulfat |
| 4,20 g | mit 1 bis 4 Ethylenoxideinheiten ethoxyliertes Dinatriumsalz des Laurylalkoholhalbesters der Sulfobernsteinsäure |
| 1,00 g | mit 10 Ethylenoxideinheiten ethoxyliertes Natriumsalz des Laurylalkoholpolyethylenglykolethercarboxylats |
| 2,00 g | eines Gemischs aus den mit 200 Ethylenoxideinheiten ethoxylierten Polyethylenglykolethern der Mono- und Diglyceride des Palmkernöls und den mit 7 Ethylenoxideinheiten ethoxylierten Polyethylenglykolethern der Kokosfettsäuremono- und -diglyceride (Rewoderm® LI S 80 der Firma Rewo Chemische Werke GmbH, Steinau/BRD) |
| 2,00 g | Lauryldimethylammoniumhydroxypropylderivat des hydrolysierten Kollagens (Lamequat®L der Firma Henkel KGaA/Düsseldorf/ BRD) |
| 0,60 g | Triethylenglykoldistearat |
| 0,20 g | Milchsäure |
| 0,30 g | Parfümöl |
| 82,70 g | Wasser |

100,00 g

**Vergleichsversuche zur Hautverträglichkeit**

Als Vergleichsversuch zur Hautverträglichkeit des erfindungsgemäßen Mittels wurde ein Test in Analogie zu der in der Literatur von P. J. Frosch, A. M. Kligman, The soap chamber test, Am. Acad. Dermatol. 1 (1979), Seiten 35 bis 41 beschriebenen Methode mit 12 Versuchspersonen über einen Zeitraum von 5 Tagen durchgeführt.

Die erfindungsgemäßen Beispiele 3 und 4 wurden hinsichtlich ihrer Hautverträglichkeit mit den nicht-erfindungsgemäßen Haar- und Körperreinigungsmitteln gemäß den Beispielen I und II, die keinen stickstoffreien Verdicker enthalten, verglichen. Die Zusammensetzungen der erfindungsgemäßen Beispiele 3 und 4 sowie der nicht-erfindungsgemäßen Beispiel I und II sind in der folgenden Tabelle 1 wiedergegeben.

Tabelle 1

| Beispiel | 3 | 4 | I | II |
|---|---|---|---|---|
| mit 1 bis 4 Ethylenoxideinheiten ethoxyliertes Natriumlaurylethersulfat | 7,00 g | 7,00 g | 7,00 g | 7,00g |
| mit 1 bis 4 Ethylenoxideinheiten ethoxyliertes Dinatriumsalz des Laurylalkoholhalbesters der Sulfobernsteinsäure | 4,20 g | 4,20 g | 4,20 g | 4,20 g |
| mit 10 Ethylenoxideinheiten ethoxyliertes Laurylalkoholpolyethylenglykolethercarboxylat | 2,80 g | - | 2,80 g | - |
| Decylglucosid (Oramix® NS 10 der Firma Seppic, Paris/Frankreich) | - | 2,80 g | - | 2,80 |
| Gemisch aus mit 200 Ethylenoxideinheiten ethoxylierten Polyethylenglykolethern der Mono- und Diglyceride des hydrierten Palmkernöls und den mit 7 Ethylenoxideinheiten ethoxylierten Polyethylenglykolethern der Kokosfettsäuremono- und diglyceride (Rewoderm® LI S 80 der Firma Rewo Chem. Werke GmbH, Steinau/BRD) | 2,00 g | 2,00 g | - | - |
| Lauryldimethylammoniumhydroxypropylderivat des Kollagens (Lamequat® L der Firma Henkel KGaA, Düsseldorf/BBRD) | 2,00 g | 2,00 g | 2,00 g | 2,00 g |
| Triethylenglykoldistearat | 0,60 g | 0,60 g | 0,60 g | 0,60 g |
| Milchsäure | 0,20 g | 0,20 g | 0,20 g | 0,20 g |
| Parfümöl | 0,30 g | 0,30 g | 0,30 g | 0,30 g |
| Wasser | 80,90 g | 80,90 g | 82,90 g | 82,90 g |
| | 100,00 g | 100,00 g | 100,00 g | 100,00 g |

Der Vergleichsversuch zur Hautverträglichkeit wurde mit Finn-Kammern, Aluminiumkammern mit 12 mm Durchmesser, durchgeführt. In die Finn-Kammern wurde zunächst jeweils ein handelsübliches Filterpapier von 11 mm Durchmesser eingelegt. Sodann wurden jeweils auf ein Filterpapier 0,05 ml jeweils einer der folgenden wäßrigen Testlösungen aufgebracht.

Als Testlösungen wurden verwendet:

(1) 8 %ige wäßrige Lösung des Haar- und Körperreinigungsmittels gemäß Beispiel 3
(2) 8 %ige wäßrige Lösung des Haar- und Körperreinigungsmittels gemäß Beispiel 4

(I) 8 %ige wäßrige Lösung des nicht-erfindungsgemäßen Haar- und Körperreinigungsmittels gemäß Beispiel I

(II) 8 %ige wäßrige Lösung des nicht-erfindungsgemäßen Haar- und Körperreinigungsmittels gemäß Beispiel II

(III) 0,2 %ige wäßrige Natriumlaurylsulfatlösung als Standardlösung

Pro Unterarm der 12 Versuchspersonen wurde jeweils 5 Finn-Kammern - pro Testlösung (1), (2), (I), (II) und (III) jeweils eine Kammer - aufgesetzt und mit handelsüblichen Heftpflaster befestigt.

Am ersten Versuchstag betrug die Kontaktzeit der Finn-Kammern auf der Haut 18 Stunden. An den folgenden vier Tagen wurden jeweils frische Testlösungen in gleicher Menge und Konzentration wie am ersten Versuchstag auf die Filterpapiere aufgebracht. Die Kontaktzeit der Finn-Kammern auf der Haut betrug am 2 bis 5 Tag des Versuchs jeweils 6 Stunden.

Die Hautreaktionen auf die Testlösungen wurde zunächt getrennt für die Erythem-, die Fissurenbildung und die Schuppung nach folgenden Bewertungssystem beurteilt:

Erythem:
- 0 negativ
- 1 sehr leichtes punktförmiges oder diffuses Erythem
- 2 gut erkennbares scharf begrenztes Erythem
- 3 mittelstarkes Erythem

Schuppung:
- 0 negativ
- 1 Trockenheit
- 2 feine Schuppung
- 3 mäßige Schuppung
- 4 starke Schuppung mit großen Flecken

Fissuren:
- 0 negativ
- 1 sehr oberflächliche epidermale Separation feine Risse
- 2 einzelne oder mehrere weite Fissuren
- 3 tiefe Fissuren mit Blutungen oder Exsudation

Bei den Testlösungen (I) und (II), die den nicht-erfindungsgemäßen Beispielen I und II entsprechen, traten zusätzlich zur Schuppung leichte Erytheme (< 1) und oberflächliche Fissuren (< 1) auf, die bei den Testlösungen 1 und 2, die den erfindungsgemäßen Beispielen 3 und 4 entsprechen, nicht beobachtet wurden.

Da die Schuppung in stärkerem Maße als die Erythem- oder Fissurenbildung auftrat, wurden nur Werte für die Schuppung berücksichtigt.

Die an beiden Unterarmen der 12 Versuchspersonen nach dem vorstehend beschriebenen Bewertungssystem erhaltenen absoluten Werte für die Schuppung durch die jeweilige Testlösung wurde zur Schuppung durch die Standardlösung (III) gemäß der folgenden Formel in Relation gesetzt:

$$\text{Schuppung, relativ} = \frac{\text{Schuppung Testlösung}}{\text{Schuppung Standardlösung (III)}}$$

Bei den in der folgenden Tabelle 2 angegebenen Zahlenwerten handelt es sich jeweils um die Mittelwerte der nach der vorstehenden Formel mit 12 Versuchspersonen ermittelten Werte für die relative Schuppung.

Tabelle 2

| Testlösung gemäß Beispiel | 3 | 4 | I | II |
|---|---|---|---|---|
| Schuppung, relativ | 0,33 | 0,41 | 0,45 | 0,62 |

Die ermittelten relativen Werte für die Schuppung zeigen, daß die erfindungsgemäßen Beispiele 3 und 4 deutlich besser hautverträglich sind, als die nicht-erfindungsgemäßen Beispiele I und II. An den mit den erfindungsgemäßen Beispielen 3 und 4 behandelten Hautpartien wurden zudem, im Gegensatz zu den mit den nicht-erfindungsgemäßen Beispielen behandelten Partien, keine Erytheme und keine Fissuren festgestellt.

Darüberhinaus wird durch den Vergleich der relativen Werte für die Schuppung der erfindungsgemäßen Beispiele 3 und 4 mit den nicht-erfindungsgemäßen Beispielen I beziehungsweise II, die sich von den erfindungsgemäßen Beispielen 3 und 4 nur durch das Fehlen des stickstofffreien Verdickers unterscheiden, der synergistische Effekt der erfindungsgemäßen Kombination des Tensidgemisches aus den Komponenten (A), (B) und (C) mit dem stickstoffreien Verdicker der Komponente (D) auf die Hautverträglichkeit deutlich.

Der Vergleichsversuch zeigt daher, daß sich die erfindungsgemäßen Haar- und Körperreinigungsmittel durch eine hervorragende Hautverträglichkeit auszeichnen.

Alle Prozentangaben dieser Anmeldung stellen, soweit nicht anders angegeben, Gewichtsprozent dar.

## Patentansprüche

1. Haar- und Körperreinigungsmittel, dadurch gekennzeichnet, daß es

(A) 1 bis 28 Gewichtsprozent mindestens eines Alkali- oder Erdalkalisalzes eines mit 1 bis 10 Ethylenoxideinheiten ethoxylierten $C_{10}$-bis $C_{18}$-Alkylethersulfats,

(B) 1 bis 28 Gewichtsprozent mindestens eines ethoxylierten Sulfobernsteinsäurehalbesters der allgemeinen Formel

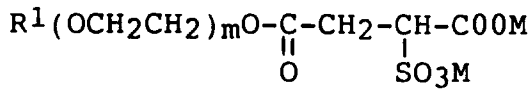 (I),

wobei $R^1$ einen $C_{10}$- bis $C_{18}$-Alkylrest bedeutet, M ein Alkali- oder Erdalkalikation darstellt und m eine ganze Zahl von 1 bis 10 bedeutet,

(C) 1 bis 28 Gewichtsprozent mindestens eines Tensids, welches ausgewählt ist aus

- einem Alkylethercarboxylat der allgemeinen Formel

$$R^2(OCH_2CH_2)_nOCH_2COOM \qquad (II),$$

wobei $R^2$ einen $C_{10}$- bis $C_{18}$-Alkylrest bedeutet, M ein Alkali- oder Erdalkalikation darstellt und n eine ganze Zahl von 1 bis 20 bedeutet, und
- einem Alkylpolyglucosid der allgemeinen Formel

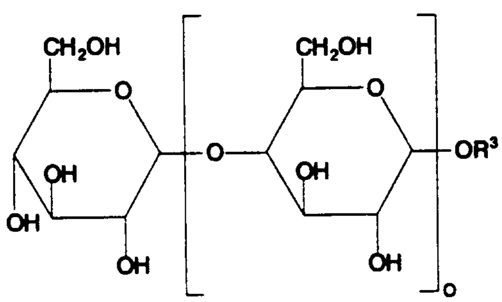 (III),

wobei $R^3$ einen linearen oder verzweigten, gesättigten oder ungesättigten $C_8$- bis $C_{18}$-Alkylrest darstellt und o eine Zahl von 1 bis 5 bedeutet sowie

(D) 0,1 bis 5 Gewichtsprozent mindestens eines stickstoffreien Verdickers, der ausgewählt ist aus

- ethoxylierten Methylglucosediestern von $C_{11}$- bis $C_{20}$-Fettsäuren der allgemeinen Formel

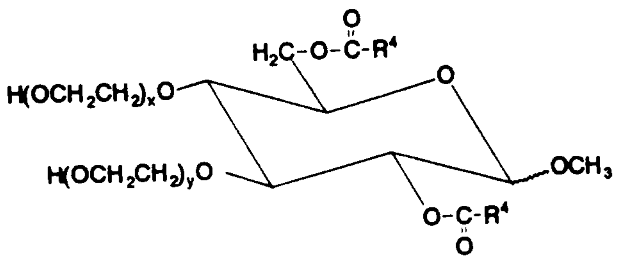 (IV),

wobei $R^4$ einen $C_{11}$- bis $C_{20}$-Alkylrest, darstellt und die Summe von x und y 50 bis 200 ergibt,

- Polyethylenglykoldiestern der allgemeinen Formel

$$R^5-\overset{\overset{O}{\|}}{C}O(CH_2CH_2O)_z-\overset{\overset{O}{\|}}{C}-R^5 \qquad (V),$$

wobei $R^5$ einen $C_{11}$- bis $C_{20}$-Alkylrest bedeutet und z eine ganze Zahl zwischen 70 und 200 darstellt und

- Gemischen aus a) mindestens einem Polyethylenglykolether von Fettsäurepartialglyceriden der allgemeinen Formel

$$R^6-\overset{\overset{O}{\|}}{C}-OCH_2-\underset{\underset{OH}{|}}{CH}-CH_2(OCH_2CH_2)_pOH \qquad (VI),$$

wobei $R^6$ einen $C_{11}$- bis $C_{20}$-Alkylrest bedeutet und p eine ganze Zahl von 100 bis 300 darstellt und b) mindestens einem Polyethylenglykolether eines Fettsäurepartialglycerids der allgemeinen Formel (VI), wobei $R^6$ einen $C_{11}$- bis $C_{20}$-Alkylrest bedeutet und p eine ganze Zahl von 5 bis 10 darstellt,

enthält, wobei das Mittel außer den Komponenten (A), (B) und (C) keine weiteren Tenside enthält.

2. Mittel nach Anspruch 1, dadurch gekennzeichnet, daß es 4 bis 10 Gewichtsprozent der Komponente (A) enthält.

3. Mittel nach einem der Ansprüche 1 oder 2, dadurch gekennzeichnet, daß es 2 bis 8 Gewichtsprozent der Komponente (B) enthält.

4. Mittel nach einem der Ansprüche 1 bis 3, dadurch gekennzeichnet, daß es 1 bis 6 Gewichtsprozent der Komponente (C) enthält.

5. Mittel nach einem der Ansprüche 1 bis 4, dadurch gekennzeichnet, daß das Gewichtsverhältnis der Komponente (A) zur Komponente (B) sowie der Komponente (A) zur Komponente (C) jeweils 1:0,1 bis 1:1 und das Gewichtsverhältnis der Komponente (A) zur Komponente (D) 1:0,1 bis 1:0,8 beträgt.

6. Mittel nach einem der Ansprüche 1 bis 5, dadurch gekennzeichnet, daß es das Tensidgemisch aus den Komponenten (A), (B) und (C) in einer Menge von 12 bis 30 Gewichtsprozent enthält.

7. Mittel nach einem der Ansprüche 1 bis 6, dadurch gekennzeichnet, daß es als Komponente (C) ein Alkylethercarboxylat der allgemeinen Formel (II) enthält.

8. Mittel nach einem der Ansprüche 1 bis 7, dadurch gekennzeichnet, daß es als Komponente (D) Gemische bestehend aus mindestens zwei Polyethylenglykolethern von Fettsäurepartialglyceriden a) und b) der allgemeinen Formel (VI) enthält.

9. Mittel nach einem der Ansprüche 1 bis 8, dadurch gekennzeichnet, daß es 65 bis 85 Gewichtsprozent Wasser enthält.

10. Mittel nach einem der Ansprüche 1 bis 9, dadurch gekennzeichnet, daß es 2 bis 20 Gewichtsprozent für kosmetische Reinigungsmittel übliche kosmetische Zusatzstoffe enthält.

## Claims

1. Hair and body cleansing agent, characterised in that it contains:

(A) 1 to 28 weight % of at least one alkali metal salt or alkaline earth metal salt of a $C_{10}$ to $C_{18}$ alkyl ethersulphate ethoxylated with 1 to 10 ethylene oxide units;

(B) 1 to 28 weight % of at least one ethoxylated sulphosuccinic acid half ester of the general formula

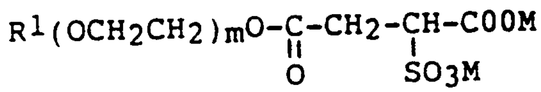

(I)

where $R^1$ represents a $C_{10}$- to $C_{18}$- alkyl residue, M represents an alkali metal or alkaline earth metal cation, and m represents an integer from 1 to 10;

(C) 1 to 28 weight % of at least one surfactant, which is selected from among

- an alkyl ether carboxylate of the general formula

$$R_2(OCH_2CH_2)_nOCH_2COOM \quad (II)$$

in which $R^2$ represents a $C_{10}$- to $C_{18}$- alkyl residue, M represents an alkali metal or alkaline earth metal cation, and n represents an integer from 1 to 20; and

- an alkylpolyglucoside of the general formula

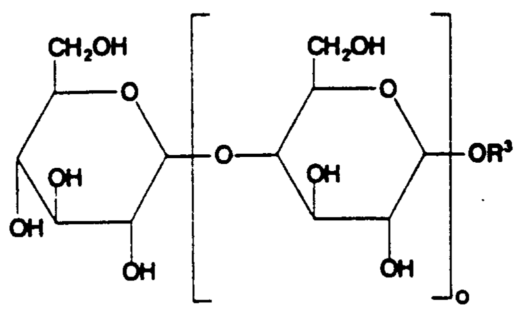

(III)

in which $R^3$ represents a linear or branched, saturated or non-saturated $C_8$- to $C_{18}$- alkyl residue and o represents an integer from 1 to 5; and

(D) 0.1 to 5 weight % of at least one nitrogen-free thickener, which is selected from among

- ethoxylated methyl glucose diesters of $C_{11}$- to $C_{20}$- fatty acids of the general formula

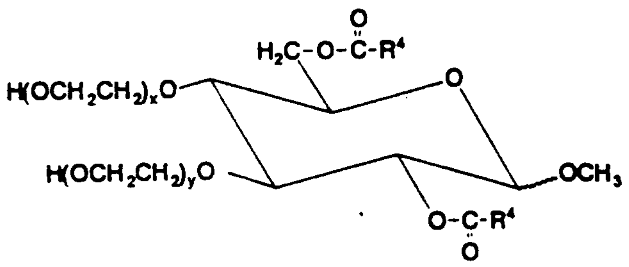

(IV)

in which $R^4$ represents a $C_{11}$- to $C_{20}$- alkyl residue, and the sum of x and y is from 50 to 200;

- polyethylene glycol diesters of the general formula

$$R^5-\overset{O}{\overset{\|}{C}}O(CH_2CH_2O)_z-\overset{O}{\overset{\|}{C}}-R^5 \qquad (V)$$

in which $R^5$ represents the $C_{11}$- to $C_{20}$-alkyl residue and z represents an integer between 70 and 200; and

- mixtures of a) at least one polyethylene glycol ether of fatty acid partial glycerides of the general formula

$$R^6-\overset{O}{\overset{\|}{C}}-OCH_2-\underset{OH}{\underset{|}{C}}H-CH_2(OCH_2CH_2)_pOH \qquad (VI)$$

in which $R^6$ represents a $C_{11}$- to $C_{20}$- alkyl residue and p is an integer from 100 to 300 and b) at least one polyethylene glycol ether of a fatty acid partial glyceride of the general formula (VI), in which $R^6$ represents a $C_{11}$- to $C_{20}$ alkyl residue and p represents an integer from 5 to 10;

in which the agent does not contain any further surfactants other than the components (A), (B) and (C).

2. Agent according to Claim 1, characterised in that it contains 4 to 10 weight % of component (A).

3. Agent according to either of Claims 1 and 2, characterised in that it contains 2 to 8 weight % of component (B).

4. Agent according to any of Claims 1 to 3, characterised in that it contains 1 to 6 weight % of component (C).

5. Agent according to any of Claims 1 to 4, characterised in that the weight ratio of component (A) to component (B) and component (A) to component (C) is 1:0.1 to 1:1 in each case, and the weight ratio of component (A) to component (D) is 1:0.1 to 1:0.8.

6. Agent according to any of Claims 1 to 5, characterised in that it contains the surfactant mixture of components (A), (B) and (C) in an amount of from 12 to 30 weight %.

7. Agent according to any of Claims 1 to 6, characterised in that it contains, as component (C), an alkylether carboxylate of the general formula (II).

8. Agent according to any of Claims 1 to 7, characterised in that it contains, as component (D), mixtures consisting of at least two polyethylene glycol ethers of fatty acid partial glycerides a) and b) of the general formula (VI).

9. Agent according to any of Claims 1 to 8, characterised in that it contains 65 to 85 weight % of water.

10. Agent according to any of Claims 1 to 9, characterised in that it contains 2 to 20 weight % of cosmetic additives which are conventional cosmetic cleansing agents.

**Revendications**

1. Agent de nettoyage corporel et capillaire, caractérisé en ce qu'il contient:

(A) de 1 à 28% en poids d'au moins un sel alcalin ou alcalino-terreux d'un alkyléther-sulfate éthoxylé en $C_{10}$-$C_{18}$ comprenant de 1 à 10 motifs oxyde d'éthylène,

(B) de 1 à 28% en poids d'au moins d'un hémi-ester d'acide sulfo succinique éthoxylé de formule générale

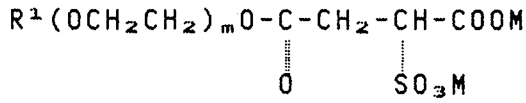 (I)

dans laquelle $R^1$ représente un reste alkyle en $C_{10}$-$C_{18}$, M un cation alcalin ou alcalino-terreux, et m un nombre entier compris entre 1 et 10.

(C) de 1 à 28% en poids d'au moins un agent tensio-actif qui est choisi parmi:

- un carboxylate d'éther-alkyle de formule générale

$$R^2(OCH_2CH_2)_nOCH_2COOM \qquad (II)$$

dans laquelle $R^2$ représente un reste alkyle en $C_{10}$-$C_{18}$, M représente un cation alcalin ou alcalino-terreux, et n un nombre entier compris entre 1 et 20, et

- un polyglucoside d'alkyle de formule générale

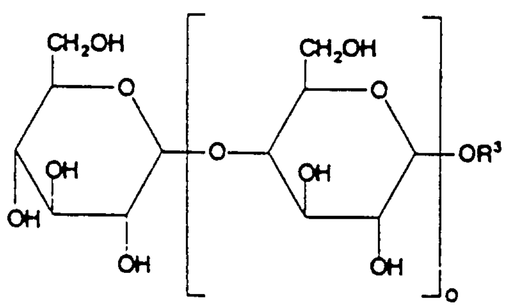 (III),

dans laquelle $R^3$ représente un reste alkyle en $C_8$-$C_{18}$ saturé ou non saturé, à chaîne droite ou ramifiée, et o un nombre allant de 1 à 5, ainsi que

(D) de 0,1 à 5% en poids d'au moins un épaississant exempt d'azote qui est choisi parmi

- des diesters de méthylglucose éthoxylés d'acides gras en $C_{11}$-$C_{20}$ de formule générale

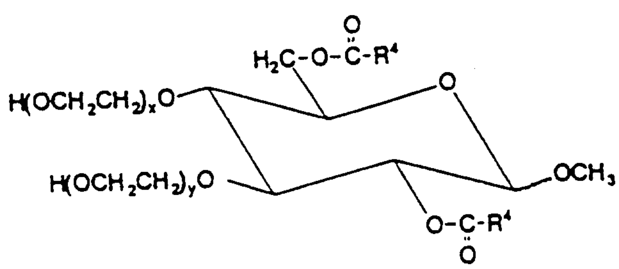 (IV),

dans laquelle $R^4$ est un reste alkyle en $C_{11}$-$C_{20}$, et la somme de x et y va de 50 à 200,

- des diesters de polyéthylèneglycol de formule générale

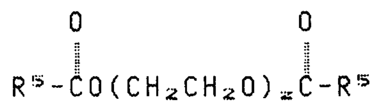

(V)

dans laquelle $R^5$ représente un reste alkyle en $C_{11}$-$C_{20}$ et z un nombre entier compris entre 70 et 200, et

- des mélanges de a) au moins un éther de polyéthylèneglycol de glycérides partiels d'acides gras de formule générale

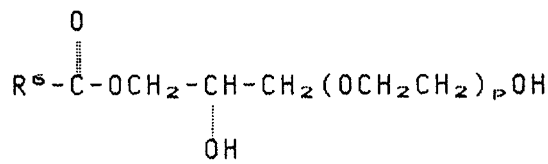

(VI)

dans laquelle $R^6$ représente un reste alkyle en $C_{11}$-$C_{20}$, et p un nombre entier allant de 100 à 300 et b) au moins un éther de polyéthylèneglycol d'un glycéride partiel d'acides gras de formule générale (VI), dans laquelle $R^6$ représente un reste alkyle en $C_{11}$-$C_{20}$ et p un nombre entier allant de 5 à 10,

l'agent ne contenant, outre les composants (A), (B) et (C), aucun autre agent tensio-actifs.

**2.** Agent selon la revendication 1, caractérisé en ce qu'il contient de 4 à 10% en poids du composant (A).

**3.** Agent selon l'une des revendications 1 ou 2, caractérisé en ce qu'il contient de 2 à 8% en poids du composant (B).

**4.** Agent selon l'une de revendications 1 à 3, caractérisé en ce qu'il contient de 1 à 6% en poids du composant (C).

**5.** Agent selon l'une des revendications 1 à 4, caractérisé en ce que le rapport pondéral du composant (A) au composant (B) ainsi que celui du composant (A) au composant (C) atteignent chacun 1:0,1 à 1:1, et le rapport pondéral du composant (A) au composant (D) va de 1:0,1 à 1:0,8.

**6.** Agent selon l'une des revendications 1 à 5, caractérisé en ce qu'il contient le mélange d'agents tensio-actifs venant des composants (A),(B) et (C) à raison de 12 à 30% en poids.

**7.** Agent selon l'une des revendications 1 à 6, caractérisé en ce qu'il contient comme composant (C) un carboxylate d'éther-alkyle de formule générale (II).

**8.** Agent selon l'une des revendications 1 à 7, caractérisé en ce qu'il contient comme composant (D) des mélanges constitués d'au moins deux éthers de polyéthylèneglycol de glycérides partiels d'acides gras a) et b) de formule générale (VI).

**9.** Agent selon l'une des revendications 1 à 8, caractérisé en ce qu'il contient de l'eau à raison de 65 à 85% en poids.

**10.** Agent sel on l'une des revendications 1 à 9, caractérisé en ce qu'il contient de 2 à 20% en poids d'additifs cosmétiques usuels pour les agents de nettoyage cosmétiques.